# EUROPEAN PATENT APPLICATION

(11) **EP 4 286 838 A1**
(43) Date of publication of application: **06.12.2023**
(21) Application number: 21923476.2
(22) Date of filing: 16.08.2021
(51) Int. Cl.: G01N 23/223

(54) **METHOD OF DETERMINING HAFNIUM CONTENT IN METALLIC ZIRCONIUM AND ALLOYS BASED THEREON**

(30) Priority: 01.02.2021 RU 2021102186
(71) Applicant: AKTSIONERNOE OBSHCHESTVO "CHEPETSKIJ MEKHANICHESKIJ ZAVOD", Glazov, 427622 (RU); Chastnoe Uchrezdenie Po Obespecheniyu Nauchnogo Razvitiya Atomnoj Otrasli "Nauka I Innovacii", Moscow, 119017 (RU)
(72) Inventor: KARAVAEVA, Olga Alekseevna, Glazov, 427628 (RU); VARKENTIN, Nikolaj Yakovlevich, Glazov, 427628 (RU)
(74) Representative: Osmans, Voldemars
(86) International application number: PCT/RU2021/050264
(87) International publication number: WO 2022/164340

(57) **Abstract**

The invention relates to the field of analytical chemistry and physical methods of analysis, and can be used to determine the hafnium content in metallic zirconium and alloys based thereon. The problem addressed by the proposed invention is to separate the overlapping lines of zirconium in the second order of reflection and hafnium. The proposed method includes plotting a calibration curve for the dependence of the fluorescence intensity of lines of hafnium on its concentration in samples with established hafnium content, preparing samples to a template, the dimensions of which correspond to the sample receptacle of a spectrometer, collimating the emission with a fine collimator with an angular divergence of 14-17 degrees, and separating the spectral range of the hafnium line using an LiF220 crystal analyzer so as to establish thresholds of an amplitude discriminator in a narrow range sufficient for cutting off impulses with high voltage generated by more highenergy zirconium quanta.

## Description

The invention relates to the analytical chemistry and physical methods of analysis and can be used to determine the content of hafnium in metallic zirconium and alloys based thereon.

The fuel assemblies of power reactors consist of zirconium components, so zirconium alloys are essential structural materials of the nuclear power industry. Due to the high similarity of physical and chemical properties, zirconium is always accompanied by an isomorphic hafnium impurity, which is an undesirable element due to a large neutron capture cross-section. This fact determines the relevance of the task for the determination of hafnium content in metallic zirconium and zirconium-based alloys. The content of hafnium in nuclear grade zirconium (according to ASTM B 349-01) and its foreign alloys and Russian alloys shall not exceed 0.01 and 0.05%, respectively.

X-ray fluorescence is one of physical methods of analysis used in the practice of analytical chemistry. This method is based on irradiation of a sample with X-rays generated by an X-ray tube anode; decomposition into a spectrum of secondary X-rays and allocation of a specified spectral interval; measurement of the intensity of the characteristic emission of an analyte followed by calculation of its content by a calibration curve for the dependence of fluorescence intensity on concentration. When using wavelength dispersive X-ray fluorescence spectrometers, the difficulty of implementing the X-ray fluorescence to determine hafnium content in materials with a zirconium matrix element is associated with the overlapping of the more intense zirconium line ZrKβ2_II in the second reflection order on the less intense hafnium line HfLβ1. Wherein, it should be noted that the greater the ratio of the mass fraction of zirconium to hafnium in the material, the stronger the overlap of lines.

The closest technical solution is the method of X-ray fluorescent determination of hafnium content in zirconium oxide [Hasany S.M., Rashid F., Rashid A. Determination of traces of hafnium in zirconium oxide by wavelength dispersive X-Ray fluorescence spectrometry / S.M. Hasany // Journal of Radioanalytical and Nuclear Chemistry. 1990. V. 142. No. 2. P.505-514.]. The measurement is performed using a wavelength dispersive X-ray fluorescence spectrometer: hafnium line HfLβ1 is extracted by LiF220 crystal analyzer, emission is collimated by a fine collimator, emission intensity is detected by a scintillation detector, hafnium content is measured by the external standard method, which involves plotting a calibration curve for the dependence of the fluorescence intensity of hafnium lines HfLβ1 on its concentration in artificial mixtures of zirconium and hafnium oxides.

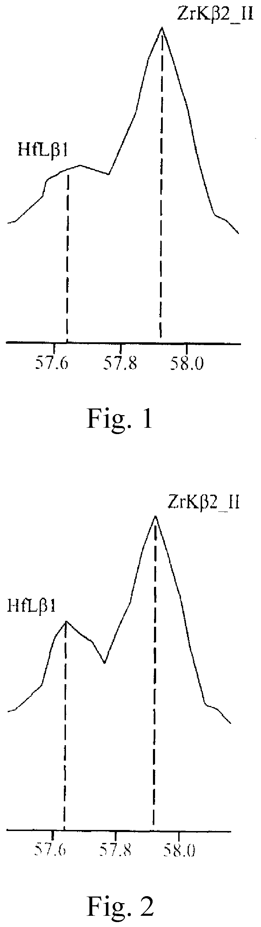

The known method is implemented for zirconium dioxide, where the stoichiometric content of zirconium matrix element does not exceed 56.2 %. Due to the suppression of hafnium analytical signal involving the overlapping of the more intense zirconium lines ZrKβ2_II in the second order of reflection on the less intense hafnium line HfLβ1, increase in the content of zirconium matrix element will lead to an inability to separate zirconium and hafnium spectrum lines and, therefore, to incorrect results (Fig. 1). Thus, the known method is not applicable to the determination of hafnium content in metallic zirconium and zirconium-based alloys, because the content of the zirconium matrix element therein is 90 to 100%.

The objective of the invention is to separate the overlapped lines of zirconium ZrKβ2_II in the second reflection order and hafnium HfLβ1 to determine hafnium content in metallic zirconium and alloys based thereon by X-ray fluorescence using a wavelength dispersive X-ray fluorescence spectrometer.

To achieve the technical result in the proposed method comprising plotting a calibration curve for the dependence of fluorescence intensity of hafnium lines HfLβ1 on its concentration in samples with the established hafnium content, preparation of a sample in a template whose dimensions correspond to the spectrometer sample receptacle, collimation of emission by a fine collimator with an angular divergence of 14-17°, separation of the spectral interval of hafnium line HfLβ1 by LiF220 crystal analyzer with setting thresholds of the amplitude discriminator in a narrow interval sufficient to cut off high-voltage pulses generated by higher-energy quanta of zirconium.

In contrast to the closest technical solution, the proposed method determines hafnium content in metallic zirconium and alloys based thereon, and the setting of pulse discriminator thresholds enables the most complete removal of an overlap from zirconium line ZrKβ2_II in the second reflection order and isolates independent hafnium line HfLβ1 (Fig. 2).

A calibration curve for the dependence of fluorescence intensity of hafnium line HfLβ1 on its concentration is plotted using samples of metallic zirconium with established hafnium content.

The calibration curve for the dependence of fluorescence intensity of HfLβ1 line on its concentration can be plotted using mixtures of zirconium and hafnium oxides with known hafnium content subject to conversion factors that take into account the difference in absorption degree of calibration material and sample material.

Sample preparation in a template is carried out by pressing chips, powder or irregularshaped pieces.

The proposed method of the determination of hafnium in metallic zirconium and alloys based thereon is implemented through the following examples.

### Example 1.

Metallic zirconium chips are pressed into a template using a press. Hafnium line HfLβ1, LiF220 crystal analyzer, and a narrow collimator were chosen in the wavelength dispersive X-ray fluorescence spectrometer settings to plot the calibration curve and to make further measurements. The amplitude discriminator threshold interval of 400-900 mV was set to cut off zirconium pulses with voltages over 900 mV (using ARL Advant'X ThermoTechno spectrometer as an example). A calibration curve for the dependence of the fluorescence intensity of hafnium line HfLβ1 on its concentration in the range of the established hafnium contents from 0.001 to 0.5 % in the metallic zirconium samples was plotted. Hafnium line HfLβ1 intensity of the sample was measured for 10-100 seconds. Hafnium content in the sample of metallic zirconium was determined using the calibration curve. Results of parallel measurements were checked using the accuracy check standard, and an average value was calculated.

### Example 2.

The difference from Example 1 is that the calibration curve for the dependence of the fluorescence intensity of hafnium line HfLβ1 on its concentration in the range of the established hafnium content from 0.001 to 0.5 % was plotted using samples from mixtures of zirconium and hafnium oxides. Then the hafnium content in the zirconium alloy sample was determined by the calibration curve subject to conversion factors that take into account the difference in the degree of fluorescence absorption of zirconium and hafnium oxide mixtures and zirconium alloy.

To check the accuracy of measurements, hafnium content was determined in reference samples of zirconium alloys with qualified hafnium content by the proposed method, the results are provided in Table 1.

**Table 1 - Results of hafnium content determination in reference samples using the proposed method**

| Reference sample | Reference sample material | Qualified content of hafnium, % | Result of determination using the proposed method |
|---|---|---|---|
| SRM 360b (NIST)^{∗} | Zirconium-tin, -iron, - chromium alloy (Zircaloy alloy) | 0.00785 ± 0.00026 | 0.0080 |
| OSO 95 1313-2011 (VNIINM JSC)^{∗} | Zirconium-niobium alloy (E110) | 0.0112 ± 0.0003 | 0.0112 |
| OSO 95 1157-2017P (VNIINM JSC)^{∗∗} | Zirconium-niobium, -tin, - iron alloy (E635) | 0.0331 ± 0.0004 | 0.0332 |

| | | | |
|---|---|---|---|
| * qualified by inductively coupled mass spectrometry and spark optical emission spectrometry ** qualified by inductively coupled plasma atomic absorption spectroscopy | | | |

The table suggests that the proposed method provides the achievement of technical result involving the possibility of hafnium content determination in metallic zirconium and alloys based thereon using X-ray fluorescence.

## Claims

1. A method of determining hafnium content in metallic zirconium and alloys based thereon comprising plotting a calibration curve for the dependence of fluorescence intensity of hafnium line HfLβ1 on its concentration in samples with the established hafnium content, pressing the analyzed sample into templates, whose dimensions correspond to the spectrometer sample receptacle, collimating emission by a fine collimator with an angular divergence of 14-17°, separating the spectral interval of hafnium line HfLβ1 by LiF220 crystal analyzer, **characterized in that** thresholds of the amplitude discriminator are set in a narrow interval sufficient to cut off high-voltage pulses generated by higher energy quanta of zirconium.

2. The method of determining hafnium content in metallic zirconium and alloys based thereon according to claim 1, **characterized in that** the calibration curve for the dependence of the fluorescence intensity of hafnium HfLβ1 line on its concentration is plotted using metallic zirconium with established hafnium content.

3. The method of determining hafnium content in metallic zirconium and alloys based thereon according to Claim 1, **characterized in that** the calibration curve for the dependence of the fluorescence intensity of hafnium line HfLβ1 on its concentration is plotted using mixtures of zirconium and hafnium oxides with established hafnium content with regard to conversion factors that take into account the difference in the degree of absorption of the calibration material and sample material.
